# EUROPÄISCHE PATENTSCHRIFT

(11) **EP 1 455 749 B1**
(45) Veröffentlichungstag und Bekanntmachung des Hinweises auf die Patenterteilung: **29.08.2012**
(21) Anmeldenummer: 02795169.8
(22) Anmeldetag: 12.12.2002
(51) Int. Cl.: A61K 8/60, A61K 8/73, A61K 8/64, A61Q 19/00, A61Q 17/04, A61Q 17/00, A61Q 19/08, A61Q 5/00, A61K 36/73

(54) **NEUE VERWENDUNGEN VON APFELKERNEXTRAKTEN IN KOSMETISCHEN ZUSAMMENSETZUNGEN**
NOVEL USE OF APPLE CORE EXTRACTS IN COSMETIC COMPOSITIONS
NOUVELLES UTILISATIONS D'EXTRAITS DE PEPINS DE POMME DANS DES PREPARATIONS COSMETIQUES

(30) Priorität: 21.12.2001 DE 10163246
(43) Veröffentlichungstag der Anmeldung: 15.09.2004
(73) Patentinhaber: Henkel AG & Co. KGaA, 40589 Düsseldorf (DE)
(72) Erfinder: PETERSOHN, Dirk, 50996 Köln (DE); SCHLOTMANN, Kordula, 40627 Düsseldorf (DE); JASSOY, Claudia, 40237 Düsseldorf (DE); WALDMANN-LAUE, Marianne, 40789 Monheim (DE); YÜCEL, Sevda, 41470 Neuss (DE)
(86) Internationale Anmeldenummer: PCT/EP2002/014122
(87) Internationale Veröffentlichungsnummer: WO 2003/053394

(56) Entgegenhaltungen:
- WO-A-03/039505
- DE-A- 10 163 246
- FR-A- 2 770 776
- US-A1- 2001 001 666
- PATENT ABSTRACTS OF JAPAN vol. 2003, no. 04 (C, 2. April 2003 (2003-04-02) & JP 2002 363025 A (NIPPON ZETTOC CO), 18. Dezember 2002 (2002-12-18) & DATABASE CAPLUS [Online] CHEMICAL ABSTRACTS SERVICE, COLUMBUS, OHIO, US;
- DATABASE CAPLUS [Online] CHEMICAL ABSTRACTS SERVICE, COLUMBUS, OHIO, US; retrieved from STN Database accession no. 2001:875796 XP002250830 & RU 2 150 263 A (OTKRYTOE AKTSIONERNOE OBSHCHESTVO KOSMETICHESKOE OB'EDINENIE "SVOBODA") 10. Juni 2000 (2000-06-10)
- DATABASE WPI Week 197822 Derwent Publications Ltd., London, GB; AN 1978-39536a XP002250831 & JP 53 044639 A (OGAWA) in der Anmeldung erwähnt
- DATABASE WPI Week 199921 Derwent Publications Ltd., London, GB; AN 1999-248450 XP002250832 & JP 11 071294 A (PIAS KK) in der Anmeldung erwähnt

## Beschreibung

Gegenstand der vorliegenden Erfindung sind neue Verwendungen von Apfelkernextrakten in topischen kosmetischen Zusammensetzungen.

Kosmetische Zusammensetzungen, die Apfelkernextrakte enthalten, sind im Stand der Technik bereits bekannt. Die japanische Offenlegungsschrift JP 11071294 A offenbart die Kombination von Apfelkern- und/oder Traubenkemextrakten mit Extrakten aus Saxifraga als Collagenaseinhibitor in kosmetischen Zusammensetzungen. Die Wirkstoffkombination beugt Hautfalten vor und mindert diese, außerdem eliminiert sie aktiven Sauerstoff.

Die japanische Offenlegungsschrift JP 53044639 A offenbart kosmetische Zusammensetzungen mit Apfelkernextrakten, die die Durchblutung anregen, die Versorgung der Haut und ihr Erscheinungsbild verbessern und aufgrund des Absorptionsmaximums bei einer Wellenlänge von 270 nm als UV-Schutzmittel dienen. Darüber hinaus ist ein entzündungshemmender Effekt offenbart. Haarpflegezubereitungen mit Apfelkernextrakt verbessern den Glanz und die Geschmeidigkeit des Haares.

Die russische Offenlegungsschrift RU 2150263 offenbart eine Nachtcreme, die einen Apfelkernextrakt in Kombination mit Tocopherylacetat und Magnesiumsulfat enthält.

Die WO 03/039505 A2 offenbart Apfelkernextrakte als Deodorant-Wirkstoff und Inhibitor für das Enzym beta-Glucuronidase, das an der körpergeruchsbildenden Schweißzersetzung beteiligt ist.

Die FR 2770776 A1 offenbart die Verwendung von Xylose und Xyloseestern, um die Basalschicht der Epidemis zu hydratisieren und/oder um die Synthese und Sekretion von Proteoglycanen und Glycosaminoglycanen durch die Keratinozyten zu stimulieren. Apfelextrakte, insbesondere Apfelsaftextrakte, sind als optionaler Wirkstoff enthalten. Die US 2001/0001666 A1 offenbart Äpfelsäure-haltige Apfelextrakte, die zur Hautexfoliation verwendet werden.

Erfindungsgemäß sind unter dem allgemein verwendeten Begriff "Haut" die Haut selbst, die Schleimhaut sowie die Hautanhangsgebilde, sofern sie lebende Zellen umfassen, insbesondere Haarfollikel, Haarwurzel, Haarzwiebel, das ventrale Epithel des Nagelbetts (Lectulus) sowie Talgdrüsen und Schweißdrüsen, zu verstehen.

Die menschliche Haut mit ihren Anhangsgebilden ist ein sehr komplex aufgebautes Organ, das aus einer Vielzahl unterschiedlicher Zelltypen besteht. Jede lebende Zelle dieses Organs ist in der Lage, auf Signale ihrer Umwelt, wie z.B. die Einwirkung topisch applizierter Kosmetika, zu reagieren. Diese Reaktionen der Zellen werden durch eine geordnete Regulation der Genexpression realisiert, so dass der Metabolismus von Zellen der Haut nicht statisch, sondern sehr dynamisch ist. Die Reaktionen der Haut auf Veränderungen der Umgebung dürfen jedoch nicht als Reaktionen einzelner, isolierter Zellen betrachtet werden. Vielmehr ist jede Zelle in ein komplexes Kommunikationsnetzwerk eingebunden. Dieses Netzwerk beinhaltet z.B. die Kommunikation zwischen Zellen der Epidermis und Zellen der Dermis. An der Kommunikation zwischen den Zellen der Haut sind Signalmoleküle, zum Beispiel Interleukine und Wachstumsfaktoren (z.B. KGF (keratinocyte growth factor), EGF (epidermal growth factor) oder FGF (fibroblast growth factor)) beteiligt.

Die Untersuchung von Wirkstoffeffekten an einzelnen, isolierten Zelltypen der Haut (z.B. Fibroblasten, Keratinozyten) kann nur einen unvollständigen Eindruck der tatsächlich im Organ ablaufenden Reaktionen liefern.

Prüfungen kosmetischer Wirkstoffe erfolgen in der Regel mit dem reinen Wirkstoff an isolierten Zellen der Haut (Fibroblasten- oder Keratinozytenkulturen). Die Untersuchung eines kosmetischen Wirkstoffs in Kombination mit einer galenischen Formulierungen ist dagegen sehr wünschenswert, da so die Gebrauchssituation des Kosmetikums simuliert wird. Interaktionen zwischen dem Wirkstoff und weiteren Komponenten der Formulierung können nicht im voraus ausgeschlossen werden. Solche Wechselwirkungen sind jedoch nur dann detektierbar, wenn eine galenische Formulierung anstelle eines isolierten Wirkstoffs geprüft wird.

Die Applikation des reinen Wirkstoffes zu Einzelzellkulturen der Haut ist neben den oben beschriebenen Problemen auch wegen der in der Einzelzellkultur fehlenden Hautbarriere kritisch. Es bleibt so die Frage unbeantwortet, ob der Wirkstoff überhaupt in der Lage ist, aus einer galenischen Formulierung die Barriere der Haut zu durchdringen und die lebenden Zellen des Organs, den eigentlichen Wirkort, zu erreichen.

Im Stand der Technik wurden solche Untersuchungen allerdings bisher noch nicht routinemäßig durchgeführt. Geeignete Hautmodellsysteme zwar schon seit längerem bekannt sind, werden aber kaum genutzt.

Die erfindungsgemäßen neuen Verwendungen von Apfelkernextrakten in topischen kosmetischen Zusammensetzungen beruhen auf überraschenden und für den Fachmann nicht vorherzusehenden Effekten, die an einem humanhomologen Ganzhautmodell mit anschließenden Untersuchungen der Genexpression und der Proteinsynthese *in vitro* ermittelt und phänomenologisch anhand von histologischen Schnitten und anhand des Hyaluronsäure-Gehaltes nachgewiesen wurden.

Das eingesetzte humanhomologe Hautmodell verfügt über ein *Stratum corneum* mit Barrierefunktion. Somit konnten Effekte von Apfelkernextrakten realitätsnah anhand einer galenischen Formulierung ermittelt werden. Dabei wurden Wechselwirkungen zwischen Inhaltsstoffen der galenischen Formulierung ebenso berücksichtigt wie Wechselwirkungen zwischen verschiedenen Zellen der Haut.

Die Wirkung einer Apfelkernextrakt-haltigen Cremeformulierung auf die Expression von 12 Genen, die unter anderem an der Zytoskelettbildung, der Ausbildung von Kommunikationsporen zwischen Hautzellen, der Zellhaftung an die extracelluläre Matrix, der Lipidsynthese in der Haut oder allgemein an der Hautalterung beteiligt sind, wurde mit Hilfe eines DNA-Chips ermittelt. Weiterhin wurde die Epidermisdicke des Hautmodells vermessen sowie sein Hyaluronsäuregehalt und die RNA-Ausbeute bestimmt.

Die Identifizierung der untersuchten Gene erfolgt über die sogenannte Swiss-Prot-Nummer. Swiss-Prot ist eine Proteinsequenz-Datenbank, die vom Schweizer Institut für Bioinformatik (SIB) und vom Europäischen Bioinformatik-Institut (EBI) entwickelt wurde.

Ein erster Gegenstand der vorliegenden Erfindung ist die nicht-therapeutische, kosmetische topische Verwendung von Apfelkernextrakten zur Steigerung der interzellulären Zellhaftung in der Haut und den Hautanhangsgebilden.

Ein weiterer Gegenstand der vorliegenden Erfindung ist die nicht-therapeutische, kosmetische topische Verwendung von Apfelkernextrakten zur Steigerung der Haftung zwischen Zellen und der extrazellulären Matrix in der Haut und den Hautanhangsgebilden. Am mit Apfelkernextrakten behandelten Ganzhautmodell konnte eine Steigerung der Expression der Keratine 1, 5, 10 und 14 (KRT 1, KRT 5, KRT 10 und KRT 14), der sogenannten Gap Junction-Proteine Connexin 26 und Connexin 43 und des Hyaluronsäure-rezeptors CD44 nachgewiesen werden. Der Zusammenhang zwischen diesen Proteinen und der mechanischen Stabilität der Haut, der Hautanhangsgebilde und des Haares wird im folgenden dargelegt.

Die Haut ist als Grenzfläche zur Umwelt besonderen mechanischen Belastungen ausgesetzt. Für die Homeostase der Haut ist es daher von Bedeutung, die Haut in ihrer mechanischen Schutzfunktion zu stärken und zu unterstützen. Die mechanische Belastbarkeit der Haut wird durch unterschiedliche biologische Funktionalitäten realisiert. So ist z.B. jede Zelle mit einem sogenannten Zytoskelett ausgerüstet, das aus Filamenten unterschiedlicher Größen besteht. Die Zellen der Epidermis enthalten insbesondere bestimmte Intermediärfilamente, sogenannte Keratine, die in Keratine des Typs I und des Typs II unterschieden werden. Die Keratine KRT 10 und KRT 14 gehören zu den Typ I-Keratinen, die Keratine KRT 1 und KRT 5 zu den Typ II-Keratinen. Je nach Differenzierungszustand der Keratinozyten synthetisieren die Zellen unterschiedliche Keratine der Typen I und II, die sich jeweils paarweise in der Zelle zusammenlagern (dimerisieren). In den basalen Keratinozyten werden die Keratine KRT5 und KRT14, die auch als Marker sich teilender Keratinozyten dienen, exprimiert und dimerisieren miteinander. In den suprabasalen Keratinozyten werden die Keratine KRT10 und KRT1, die auch als Marker sich differenzierender Keratinozyten dienen, exprimiert und dimerisieren ebenfalls miteinander. Durch die Steigerung der Expression der genannten Keratine wird das Zytoskelett verstärkt, das zur mechanischen Stabilität beiträgt. Die gleichzeitig gesteigerte Anzahl und Differenzierung der Keratinozyten trägt ebenfalls zur mechanischen Stabilität bei.

Cremeformulierungen mit Apfelkernextrakten steigerten die Expression der Keratine KRT5 und KRT14 um den Faktor 2,3 beziehungsweise 2,4. Die Expression der Keratine KRT10 und KRT1 wurde durch Cremeformulierungen mit Apfelkernextrakten um den Faktor 3,4 beziehungsweise 3,5 gesteigert.

Die Steigerung der Keratinozytenzahl wurde bestätigt durch die am Hautmodell nachgewiesene Steigerung der RNA-Gesamtausbeute und durch die ebenfalls nachgewiesene Zunahme der Epidermisdicke. Hautmodelle, die mit Apfelkernextrakt-haltigen Cremeformulierungen behandelt worden waren, wiesen nach sechs und 48 Stunden Inkubation eine gesteigerte RNA-Gesamtausbeute auf. Dies deutet darauf hin, dass Apfelkernextrakte einen generellen Einfluss auf den Zellmetabolismus der Hautmodelle ausüben und diesen steigern. Der Begriff "Metabolismus" wird in diesem Zusammenhang im Sinne einer Steigerung der Proliferation und der Proteinsynthese verwendet, nicht im Sinne eines Stoffwechselmetabolismus.

Die mechanische Stabilität der Haut und der Hautanhangsgebilde wird weiterhin unterstützt durch die Ausbildung von sogenannten "Gap Junctions".

Wie bereits erläutert, sind die Zellen der Haut nicht als einzelne isolierte Einheiten zu betrachten. Vielmehr sind Hautzellen in ein komplexes Kommunikationsnetzwerk eingebunden. Einen Teil dieses Netzwerkes machen die so genannten "Gap Junctions" zwischen zwei Zellen aus. Es handelt sich hierbei um Strukturen in der Zellmembran, die zur Ausbildung offener Kanäle zu benachbarten Zellen dienen. Über diese Kanäle können niedermolekulare Signalstoffe von einer Zelle zur nächsten gelangen und so Kommunikation vermitteln. An der Ausbildung der "Gap Junctions" sind bestimmte Proteine, die sogenannten Connexine, beteiligt. Die Behandlung von Hautmodellen mit Apfelkernextrakten resultierte in einer gesteigerten Expression der Gene für Connexin 43 und Connexin 26, zwei Proteinen, die auch in der Haut bekanntermaßen "Gap Junctions" bilden und somit an der Ausbildung von Kommunikationsporen zwischen Hautzellen beteiligt sind. Die "Gap Junctions" sorgen aber nicht nur für eine verbesserte Zellkommunikation auf der chemischen Ebene, sondern erhöhen auch die interzelluläre Zellhaftung in der Haut und den Hautanhangsgebilden.

Ein weiterer Gegenstand der vorliegenden Erfindung ist die nicht-therapeutische, kosmetische topische Verwendung von Apfelkernextrakten zur Steigerung der Lipidproduktion in der Epidermis.

Neben den Elementen des Zytoskeletts und den Gap Junction-Proteinen vermitteln weitere Proteine dem Hautgewebe mechanische Stabilität. Diese Proteine vermitteln eine Bindung zwischen Zellen und extrazellulärer Matrix (ECM). Bestandteil der ECM ist z. B. das Kollagen oder auch die Hyaluronsäure. Für die Bindung von Zellen an die Proteine der ECM sind Rezeptoren verantwortlich, die auf der Zelloberfläche lokalisiert sind. So bindet z.B. der Oberflächenrezeptor CD44, dessen Expression durch Apfelkernextrakte gesteigert wird, an Hyaluronsäure.

Ein weiterer Gegenstand der vorliegenden Erfindung ist die nicht-therapeutische, kosmetische topische Verwendung von Apfelkernextrakten zur Steigerung der Ausbildung von interzellulären Kommunikationsporen in der Haut und den Hautanhangsgebilden und zur Verbesserung der interzellulären Zellkommunikation.

Die erfindungsgemäße Verwendung von Apfelkernextrakten zur Steigerung der interzellulären Zellhaftung in der Haut und den Hautanhangsgebilden und zur Steigerung der Haftung zwischen Zellen und der extrazellulären Matrix in der Haut und den Hautanhangsgebilden beschränkt sich nicht auf die lebenden Zellen der Haut und der vorstehend definierten Hautanhangsgebilde, sondern auch auf das Haar einschließlich der toten Zellen des Haarschaftes. Der Entstehungsort der Haare ist in der Haut der Haarfollikel, der aus verschiedenen spezialisierten Epithelial- und Bindegewebszellen besteht. Haarfollikel durchlaufen verschiedene Wachstumphasen, wobei in der anagenen Phase Haare produziert werden. In der catagenen Phase dagegen verringert sich die Haarproduktion, der Haarfollikel verkümmert, und schließlich wird die Haarproduktion eingestellt. Der Haarfollikel ist dann im Ruhezustand bzw. in der sogenannten telogenen Phase. Während der anagenen Phase des Haarfollikels synthetisieren bestimmte Zellschichten im Haarfollikel epitheliale Keratine. Die Keratine 1 und 10 zum Beispiel kommen auch im Haarfollikel an der "Inneren Wurzelscheide" (an der Cutikula und dem "Huxley's Layer") vor. Des weiteren produzieren die Zellen der "äußeren Wurzelscheide" die Keratine 5 und 14. Epidermale Zellen, also ebenso die Keratinozyten der "Inneren" und "Äusseren Wurzelscheide", reagieren auf die Behandlung mit Apfelkernextrakten mit der verstärkten Produktion der Keratine 1, 10, 5 und 14. Es ist anzunehmen, dass es gleichzeitig zu einer verstärkten Produktion der haarspezifischen Keratine kommt. Diese Reaktion geht mit einer Erhöhung der Keratinozytenzahl einher, ein Effekt, der für die Stabilität des gesamten Haares, also auch des toten Haarschaftes, von Vorteil ist.

Am mit Apfelkernextrakten behandelten Ganzhautmodell konnte eine Steigerung der Expression des Enzyms FAS (Fatty Acid Synthase) und des Fatty Acid Binding Proteins (FABE) nachgewiesen werden. Der Zusammenhang zwischen diesen Proteinen und der körpereigenen Barrierefunktion der Haut und der Hautanhangsgebilde wird im folgenden dargelegt.

Epidermale Keratinozyten produzieren und sekretieren Lipide, um z.B. die Barrierefunktion der Haut aufrecht zu halten. Die Zusammensetzung der Lipide in den lebenden Zellschichten der Epidermis ist vergleichbar mit der Lipidzusammensetzung anderer Epithelien: Phospholipide und Cholesterol als Hauptbestandteil zellulärer Membranbilayer und Triglyceride als Energieträger sind die überwiegenden Lipide der Epidermis. Im Stratum granulosum, einer bestimmten Schicht der Epidermis, werden Lipide sehr dicht in sogenannte Granula gepackt. Während der terminalen Differenzierung zum Stratum corneum, der Hornschicht der Epidermis, werden diese Granula in den extrazellulären Raum sekretiert, wo sie dann zwischen den Corneozyten multiple Lipidbilayer formen. Während der terminalen Differenzierung der Keratinozyten verändert sich deren Lipidkomposition drastisch. So nimmt im Laufe der Differenzierung der Phospholipidgehalt ab. Das Stratum corneum schließlich ist nahezu frei von Phospholipiden, während der Anteil an Ceramiden, Sterolen und freien Fettsäuren zunimmt.

An der Biosynthese freier Fettsäuren ist das Enzym FAS (Fatty Acid Synthase) beteiligt, dessen Expression, wie oben beschrieben, durch Apfelkernextrakte gesteigert wird. Es handelt sich dabei um ein relativ großes Protein, bestehend aus über 2500 Aminosäuren, mit einem Molekulargewicht von mehr als 273 Kilo-Dalton (kDa). FAS katalysiert die Produktion langkettiger freier Fettsäuren aus Acetyl-CoA, Malonyl-CoA und NADPH. Immunhistochemische Untersuchungen haben bereits in der Vergangenheit gezeigt, dass FAS im Stratum granulosum stark und in den oberen Schichten des Stratum spinosum moderat produziert wird.

Neben der Biosynthese der freien Fettsäuren ist deren Transport in der Zelle ebenso von biologischer Bedeutung. In der Epidermis wurde unter anderem das Protein FABE (Fatty Acid Binding Protein in Epidermis) beschrieben, das freie Fettsäuren bindet und an deren Transport beteiligt zu sein scheint. FABE, dessen Expression, wie oben beschrieben, durch Apfelkernextrakte gesteigert wird, bindet bevorzugt C18-Fettsäuren, wobei mit zunehmender Anzahl an Doppelbindungen oder einer Verkürzung der Kettenlänge der Fettsäuren die Bindungsaffinität geringer wird.

Für die chemischen Prozesse, die in die körpereigene Barrierefunktion der Haut involviert sind, ist die Zellkommunikation durch interzelluläre Kommunikationsporen, die "Gap Junctions", unerlässlich.

Ein weiterer Gegenstand der vorliegenden Erfindung ist die nicht-therapeutische, kosmetische topische Verwendung von Apfelkernextrakten zur Steigerung der Expression des Keratins KRT5 mit der Swiss-Prot-Nummer P13647, des Keratins KRT14 mit der Swiss-Prot-Nummer P02533, des Keratins KRT10 mit der Swiss-Prot-Nummer P13645, des Keratins KRT1 mit der Swiss-Prot-Nummer P04264, des Gap Junction Proteins Beta 2 (Connexin 26, CXB2) mit der Swiss-Prot-Nummer P29033, des Gap Junction Proteins Alpha 1 (Connexin 43) mit der Swiss-Prot-Nummer P17302, des Hyaturonsäure-Rezeptors CD44 mit der Swiss-Prot-Nummer P16070, der Fatty Acid Synthase (FAS) mit der Enzymklassifizierung EC 2.3.1.85 und der Swiss-Prot-Nummer P49327, des Epidermal Fatty Acid Binding Proteins FABE mit der Swiss-Prot-Nummer Q01469, des Proteins PSMD2 mit der Swiss-Prot-Nummer Q13200 und des DNA binding protein A

(DBPA) mit der Swiss-Prot-Nummer P16989 in Zellen der Haut und der Hautanhangsgebilde.

Ein weiterer Gegenstand der vorliegenden Erfindung ist die nicht-therapeutische, kosmetische topische Verwendung von Apfelkernextrakten zur Inhibierung der Expression des Bone Proteoglycan II Precursors (PGS2) mit der Swiss-Prot-Nummer P07585 in Zellen der Haut und der Hautanhangsgebilde.

Am mit Apfelkernextrakten behandelten Ganzhautmodell konnte eine Inhibierung der Expression von PGS2 (Decorin) nachgewiesen werden, das, wie in Voruntersuchungen gefunden worden war, in gealterter Haut im Vergleich zu junger Haut verstärkt exprimiert wird. Die Proteine CD44 (Hyaluronsäure-Rezeptor), CXB2 (Connexin 26), PSMD2 (Swiss-Prot-Nummer Q13200), DBPA (Swiss-Prot-Nummer P16989) und FAS (Swiss-Prot-Nummer P49327), die in gealterter Haut gegenüber junger Haut verringert exprimiert sind, wurden am Ganzhautmodell nachweislich durch die Behandlung mit Apfelkernextrakten aktiviert. Somit verändert die topische Behandlung der Haut mit Apfelkernextrakten ein Genexpressionsprofil, bestehend aus sechs Altersmarkern der Haut, in die Richtung eines Genexpressionsprofils von jüngerer Haut.

Ein erfindungsgemäß besonders bevorzugter Apfelkernextrakt ist das Handelsprodukt Ederline des Herstellers Seporga. Ederline enthält Phytohormone, Isoflavonoide, Phytosterole, Triterpenoide, Tocopherole und natürliche Wachse. In vitro-Tests mit kultivierten Hautzellen zeigen laut Herstellerangaben Effekte auf die Synthese von Kollagen Typ I und Kollagen Typ III sowie auf Fibronectin. Desweiteren wurde in Probandenstudien eine positive Wirkung von Ederline auf das Hautrelief gemessen. Außerdem machen die Herstellerangaben antioxidative und antiinflammatorische Wirkungen geltend.

Die experimentellen Untersuchungen mit Ederline sind an isolierten Zellen der Haut durchgeführt worden. Beschriebene positive Effekte von Ederline auf Kollagen Typ I und III und Fibronectin konnten jedoch am Ganzhautmodell nicht bestätigt werden. Es wird daher vermutet, dass die an Einzelzellen gewonnenen Erkenntnisse hier nicht auf das Hautorgan in seiner dreidimensionalen Struktur zu übertragen sind.

Das Produkt Ederline ist einmal in wasserlöslicher Form als Ederline-H (INCI: PEG-40 Hydrogenated Castor Oil, PPG-2-Ceteareth-9, Pyrus Malus (Apple) Fruit Extract), zum anderen in fettlöslicher Form als Ederline-L (INCI: Hexyldecanol, Pyrus Malus (Apple) Fruit Extract) erhältlich.

Erfindungsgemäß geeignete Mengen an Ederline sind 0,1 - 10 Gew.-%, bevorzugt 1 - 8 Gew.-% und besonders bevorzugt 3 - 5 Gew.-%, jeweils bezogen auf die gesamte Zusammensetzung. Bezogen auf den Gehalt an aktiven Wirkstoffen, werden Apfelkernextrakte erfindungsgemäß in Mengen von 0,001 - 2 Gew.-%, bevorzugt 0,01 - 1,6 Gew.-% und besonders bevorzugt 0,03 - 1 Gew.-%, jeweils bezogen auf die gesamte Zusammensetzung, eingesetzt.

Erfindungsgemäß verwendete topische kosmetische Apfelkemextrakt-haltige Zusammensetzungen können mindestens einen organischen oder mineralischen oder modifizierten mineralischen Lichtschutzfilter enthalten. Bei den Lichtschutzfiltern handelt es sich um bei Raumtemperatur flüssig oder kristallin vorliegende Substanzen, die in der Lage sind, ultraviolette Strahlen zu absorbieren und die aufgenommene Energie in Form längerwelliger Strahlung, z. B. Wärme, wieder abzugeben. Man unterscheidet UVA-Filter und UVB-Filter. Die UVA- und UVB-Filter können sowohl einzeln als auch in Mischungen eingesetzt werden. Der Einsatz von Filtermischungen ist erfindungsgemäß bevorzugt. Die erfindungsgemäß verwendeten organischen UV-Filter sind ausgewählt aus den Derivaten von Dibenzoylmethan, Zimtsäureestern, Diphenylacrylsäureestern, Benzophenon, Campher, p-Aminobenzoesäureestern, o-Aminobenzoesäureestern, Salicylsäureestern, Benzimidazolen, 1,3,5-Triazinen, monomeren und oligomeren 4,4-Diarylbutadiencarbonsäureestern und -carbonsäureamiden, Ketotricyclo(5.2.1.0)decan, Benzalmalonsäureestern sowie beliebigen Mischungen der genannten Komponenten. Die organischen UV-Filter können öllöslich oder wasserlöslich sein. Erfindungsgemäß besonders bevorzugte öllösliche UV-Filter sind 1-(4-tert.-Butylphenyl)-3-(4'methoxyphenyl)propan-1,3-dion (Parsol^{®} 1789), 1-Phenyl-3-(4'-isopropylphenyl)-propan-1,3-dion, 3-(4'-Methylbenzyliden)-D,L-campher, 4-(Dimethylamino)-benzoesäure-2-ethylhexylester, 4-(Dimethylamino)benzoesäure-2-octylester, 4-(Dimethylamino)-benzoesäureamylester, 4-Methoxyzimtsäure-2-ethylhexylester, 4-Methoxyzimtsäurepropylester, 4-Methoxyzimtsäureisopentylester, 2-Cyano-3,3-phenylzimtsäure-2-ethylhexylester (Octocrylene), Salicyisäure-2-ethylhexylester, Salicylsäure-4-isopropylbenzylester, Salicylsäurehomomenthylester (3,3,5-Trimethyl-cyclohexylsalicylat), 2-Hydroxy-4-methoxybenzophenon, 2-Hydroxy-4-methoxy-4'-methylbenzophenon, 2,2'-Dihydroxy-4-methoxybenzophenon, 4-Methoxybenzmalonsäuredi-2-ethylhexylester, 2,4,6-Trianilino-(p-carbo-2'-ethyl-1'-hexyloxy)-1,3,5-triazin (Octyl Triazone) und Dioctyl Butamido Triazone (Uvasorb^{®} HEB) sowie beliebige Mischungen der genannten Komponenten.

Bevorzugte wasserlösliche UV-Filter sind 2-Phenylbenzimidazol-5-sulfonsäure und deren Alkali-, Erdalkali-, Ammonium-, Alkylammonium-, Alkanolammonium- und Glucammoniumsalze, Sulfonsäurederivate von Benzophenonen, vorzugsweise 2-Hydroxy-4-methoxybenzophenon-5-sulfonsäure und ihre Salze, Sulfonsäurederivate des 3-Benzylidencamphers, wie z. B. 4-(2-Oxo-3-bornylidenmethyl)benzolsulfonsäure und 2-Methyl-5-(2-oxo-3-bornyliden)sulfonsäure und deren Salze.

Bei den erfindungsgemäß bevorzugten anorganischen Lichtschutzpigmenten handelt es sich um feindisperse Metalloxide und Metallsalze, beispielsweise Titandioxid, Zinkoxid, Eisenoxid, Aluminiumoxid, Ceroxid, Zirkoniumoxid, Silicate (Talk) und Bariumsulfat. Die Partikel sollten dabei einen mittleren Durchmesser von weniger als 100 nm, vorzugsweise zwischen 5 und 50 nm und insbesondere zwischen 15 und 30 nm aufweisen, sogenannte Nanopigmente. Sie können eine sphärische Form aufweisen, es können jedoch auch solche Partikel zum Einsatz kommen, die eine ellipsoide oder in sonstiger Weise von der sphärischen Gestalt abweichende Form besitzen. Die Pigmente können auch oberflächenbehandelt, d.h. hydrophilisiert oder hydrophobiert vorliegen. Typische Beispiele sind gecoatete Titandioxide, wie z. B. Titandioxid T 805 (Degussa) oder Eusolex^{®} T2000 (Merck). Als hydrophobe Coatingmittel kommen dabei vor allem Silicone und dabei speziell Trialkoxyoctylsilane oder Simethicone in Frage. Besonders bevorzugt sind Titandioxid und Zinkoxid.

In den erfindungsgemäßen Zusammensetzungen sind die Lichtschutzfilter in Mengen von 0,1 - 30 Gew.-%, bevorzugt 1 - 20 Gew.% und besonders bevorzugt 2 - 15 Gew.-%, jeweils bezogen auf die gesamte Zusammensetzung, enthalten.

Erfindungsgemäß verwendete topische kosmetische Apfelkernextrakt-haltige Zusammensetzungen können mindestens ein Proteinhydrolysat oder dessen Derivat enthalten. Erfindungsgemäß können sowohl pflanzliche als auch tierische Proteinhydrolysate eingesetzt werden. Tierische Proteinhydrolysate sind z. B. Elastin-, Collagen-, Keratin-, Seiden- und Milcheiweiß-Proteinhydrolysate, die auch in Form von Salzen vorliegen können. Erfindungsgemäß bevorzugt sind pflanzliche Proteinhydrolysate, z. B. Soja-, Weizen-, Mandel-, Erbsen-, Kartoffel- und Reisproteinhydrolysate. Entsprechende Handelsprodukte sind z. B. DiaMin^{®} (Diamalt), Gluadin^{®} (Cognis), Lexein^{®} (Inolex) und Crotein^{®} (Croda).

Wenngleich der Einsatz der zusätzlichen Proteinhydrolysate als solche bevorzugt ist, können an ihrer Stelle gegebenenfalls auch anderweitig erhaltene Aminosäuregemische oder einzelne Aminosäuren wie beispielsweise Arginin, Lysin, Histidin oder Pyroglutaminsäure eingesetzt werden. Ebenfalls möglich ist der Einsatz von Derivaten der Proteinhydrolysate, z. B. in Form ihrer Fettsäure-Kondensationsprodukte. Entsprechende Handelsprodukte sind z. B. Lamepon^{®} (Cognis), Gluadin^{®} (Cognis), Lexein^{®} (Inolex), Crolastin^{®} oder Crotein^{®} (Croda).

Erfindungsgemäß einsetzbar sind auch kationisierte Proteinhydrolysate, wobei das zugrunde liegende Proteinhydrolysat vom Tier, von der Pflanze, von marinen Lebensformen oder von biotechnologisch gewonnenen Proteinhydrolysaten stammen kann. Bevorzugt sind kationische Proteinhydrolysate, deren Proteinanteil ein Molekulargewicht von 100 bis zu 25000 Dalton, bevorzugt 250 bis 5000 Dalton aufweist. Weiterhin sind unter kationischen Proteinhydrolysaten quaternierte Aminosäuren und deren Gemische zu verstehen. Die kationischen Proteinhydrolysate können auch noch weiter derivatisiert sein. Als typische Beispiele für erfindungsgemäß verwendete kationische Proteinhydrolysate und -derivate seien einige der im Handel erhältlichen Produkte unter ihren INCI-Bezeichnungen aufgeführt: Cocodimonium Hydroxypropyl Hydrolyzed Collagen, Cocodimonium Hydroxypropyl Hydrolyzed Casein, Steardimonium Hydroxypropyl Hydrolyzed Collagen, Steardimonium Hydroxypropyl Hydrolyzed Hair Keratin, Lauryldimonium Hydroxypropyl Hydrolyzed Keratin, Cocodimonium Hydroxypropyl Hydrolyzed Rice Protein, Cocodimonium Hydroxypropyl Hydrolyzed Silk, Cocodimonium Hydroxypropyl Hydrolyzed Soy Protein, Cocodimonium Hydroxypropyl Hydrolyzed Wheat Protein, Cocodimonium Hydroxypropyl Silk Amino Acids, Hydroxypropyl Arginine Lauryl/Myristyl Ether HCl, Hydroxypropyltrimonium Gelatin. Ganz besonders bevorzugt sind die kationischen Proteinhydrolysate und -derivate auf pflanzlicher Basis.

In den erfindungsgemäß verwendeten Zusammensetzungen sind die Proteinhydrolysate und deren Derivate in Mengen von 0,01 - 10 Gew.-%, bevorzugt 0,1 - 5 Gew.% und besonders bevorzugt 0,1 - 3 Gew.-%, jeweils bezogen auf die gesamte Zusammensetzung, enthalten.

Erfindungsgemäß verwendete topische kosmetische Apfelkernextrakt-haltige Zusammensetzungen können weiterhin mindestens ein Mono-, Oligo- oder Polysaccharid oder deren Derivate enthalten, wobei Saccharide, die Aminozucker-Bausteine enthalten, ausgeschlossen sind.

Erfindungsgemäß geeignete Monosaccharide sind z. B. Glucose, Fructose, Galactose, Arabinose, Ribose, Xylose, Lyxose, Allose, Altrose, Mannose, Gulose, Idose und Talose sowie die Desoxyzucker Fucose und Rhamnose. Bevorzugt sind Glucose, Fructose, Galactose, Arabinose und Fucose; Glucose ist besonders bevorzugt.

Erfindungsgemäß geeignete Oligosaccharide sind aus zwei bis zehn Monosaccharideinheiten zusammengesetzt, z. B. Saccharose, Lactose oder Trehalose. Ein besonders bevorzugtes Oligosaccharid ist Saccharose. Ebenfalls besonders bevorzugt ist die Verwendung von Honig, der überwiegend Glucose und Saccharose enthält. Erfindungsgemäß geeignete Polysaccharide sind aus mehr als zehn Monosaccharideinheiten zusammengesetzt. Bevorzugte Polysaccharide sind die aus α-D-Glucose-Einheiten aufgebauten Stärken sowie Stärkeabbauprodukte wie Amylose, Amylopektin und Dextrine. Erfindungsgemäß besonders vorteilhaft sind chemisch und/oder thermisch modifizierte Stärken, z. B. Hydroxypropylstärkephosphat, Dihydroxypropyldistärkephosphat oder die Handelsprodukte Dry Flo^{®}. Weiterhin bevorzugt sind Dextrane sowie ihre Derivate, z. B. Dextransulfat. Ebenfalls bevorzugt sind nichtionische Cellulose-Derivate, wie Methylcellulose, Hydroxypropylcellulose, Hydroxypropylmethylcellulose oder Hydroxyethylcellulose, sowie kationische Cellulose-Derivate, z. B. die Handelsprodukte Celquat^{®} und Polymer JR^{®}, und bevorzugt Celquat^{®} H 100, Celquat^{®} L 200 und Polymer JR^{®} 400 (Polyquaternium-10) sowie Polyquaternium-24. Weitere bevorzugte Beispiele sind Polysaccharide aus Fucose-Einheiten, z. B. das Handelsprodukt Fucogel^{®}.

In den erfindungsgemäß verwendeten Zusammensetzungen sind die Mono-, Oligo- oder Polysaccharide oder deren Derivate in Mengen von 0,1 - 10 Gew.-%, bevorzugt 0,5 - 5 Gew.% und besonders bevorzugt 1,0 - 3 Gew.-%, jeweils bezogen auf die gesamte Zusammensetzung, enthalten.

Erfindungsgemäß verwendete topische kosmetische Apfelkemextrakt-haltige Zusammensetzungen können weiterhin mindestens eine α-Hydroxycarbonsäure oder α-Ketocarbonsäure oder deren Ester-, Lacton- oder Salzform enthalten. Geeignete α-Hydroxycarbonsäuren oder α-Ketocarbonsäuren sind ausgewählt aus Milchsäure, Weinsäure, Citronensäure, 2-Hydroxybutansäure, 2,3-Dihydroxypropansäure, 2-Hydroxypentansäure, 2-Hydroxyhexansäure, 2-Hydroxyheptansäure, 2-Hydroxyoctansäure, 2-Hydroxydecansäure, 2-Hydroxydodecansäure, 2-Hydroxytetradecansäure, 2-Hydroxyhexadecansäure, 2-Hydroxyoctadecansäure, Mandelsäure, 4-Hydroxymandelsäure, Äpfelsäure, Erythrarsäure, Threarsäure, Glucarsäure, Galactarsäure, Mannarsäure, Gularsäure, 2-Hydroxy-2-methylbernsteinsäure, Gluconsäure, Brenztraubensäure, Glucuronsäure und Galacturonsäure. Die Ester der genannten Säuren sind ausgewählt aus den Methyl-, Ethyl-, Propyl-, Isopropyl-, Butyl-, Amyl-, Pentyl-, Hexyl-, 2-Ethylhexyl-, Octyl-, Decyl-, Dodecyl- und Hexadecylestern. Die α-Hydroxycarbonsäuren oder α-Ketocarbonsäuren oder ihre Derivate sind vorzugsweise in Mengen von 0,1 - 10 Gew.-%, bevorzugt 0,5 - 5 Gew.-%, jeweils bezogen auf die gesamte Zusammensetzung, enthalten.

Vorteilhafterweise können die erfindungsgemäß verwendeten Zusammensetzungen mindestens ein synthetisches filmbildendes, emulsionsstabilisierendes, verdickendes oder adhäsives Polymer, ausgewählt aus Polymeren, die kationisch, anionisch, amphoter geladen oder nichtionisch sein können, enthalten.

Erfindungsgemäß bevorzugt sind kationische, anionische sowie nichtionische Polymere. Unter den kationischen Polymeren bevorzugt sind Polysiloxane mit quaternären Gruppen, z. B. die Handelsprodukte Q2-7224 (Dow Corning), Dow Corning^{®} 929 Emulsion (mit Amodimethicone), SM-2059 (General Electric), SLM-55067 (Wacker) sowie Abil^{®}-Quat 3270 und 3272 (Th. Goldschmidt).

Bevorzugte anionische Polymere enthalten Carboxylat- und/oder Sulfonatgruppen und als Monomere zum Beispiel Acrylsäure, Methacrylsäure, Crotonsäure, Maleinsäureanhydrid und 2-Acrylamido-2-methylpropansulfonsäure. Dabei können die sauren Gruppen ganz oder teilweise als Natrium-, Kalium-, Ammonium-, Mono- oder Triethanolammonium-Salz vorliegen. Bevorzugte Monomere sind 2-Acrylamido-2-methylpropansulfonsäure und Acrylsäure. Ganz besonders bevorzugte anionische Polymere enthalten als alleiniges Monomer oder als Comonomer 2-Acrylamido-2-methylpropansulfonsäure, wobei die Sulfonsäuregruppe ganz oder teilweise in Salzform vorliegen kann. Innerhalb dieser Ausführungsform ist es bevorzugt, Copolymere aus mindestens einem anionischen Monomer und mindestens einem nichtionischen Monomer einzusetzen. Bezüglich der anionischen Monomere wird auf die oben aufgeführten Substanzen verwiesen. Bevorzugte nichtionogene Monomere sind Acrylamid, Methacrylamid, Acrylsäureester, Methacrylsäureester, Vinylpyrrolidon, Vinylether und Vinylester. Bevorzugte anionische Copolymere sind Acrylsäure-Acrylamid-Copolymere sowie insbesondere Polyacrylamidcopolymere mit Sulfonsäuregruppen-haltigen Monomeren. Ein besonders bevorzugtes anionisches Copolymer besteht aus 70 bis 55 Mol-% Acrylamid und 30 bis 45 Mol-% 2-Acrylamido-2-methylpropansulfonsäure, wobei die Sulfonsäuregruppen ganz oder teilweise als Natrium-, Kalium-, Ammonium-, Mono- oder Triethanolammonium-Salz vorliegen. Dieses Copolymer kann auch vernetzt vorliegen, wobei als Vernetzungsagentien bevorzugt polyolefinisch ungesättigte Verbindungen wie Tetraallyloxyethan, Allylsucrose, Allylpentaerythrit und Methylen-bisacrylamid zum Einsatz kommen. Ein solches Polymer ist in dem Handelsprodukt Sepigel^{®}305 der Firma SEPPIC enthalten. Die Verwendung dieses Compounds hat sich im Rahmen der erfindungsgemäßen Lehre als besonders vorteilhaft erwiesen. Auch die unter den Bezeichnungen Simulgel^{®}600, Simulgel^{®}NS und Simulgel^{®}EG als Compound mit Isohexadecan bzw. Squalan und Polysorbate-80 bzw. Polysorbate-60 vertriebenen Natriumacryloyldimethyltaurat-Copolymere mit Acrylamid bzw. Hydroxyethylacrylat bzw. Natriumacrylat haben sich als erfindungsgemäß besonders wirksam erwiesen.

Weitere besonders bevorzugte anionische Homo- und Copolymere sind unvernetzte und vernetzte Polyacrylsäuren. Dabei können Allylether von Pentaerythrit, von Sucrose und von Propylen bevorzugte Vernetzungsagentien sein. Solche Verbindungen sind zum Beispiel die Handelsprodukte Carbopol^{®}. Ein besonders bevorzugtes anionisches Copolymer enthält als Monomer zu 80 - 98 % eine ungesättigte, gewünschtenfalls substituierte C₃₋₆-Carbonsäure oder ihr Anhydrid sowie zu 2 - 20 % gewünschtenfalls substituierte Acrylsäureester von gesättigten C₁₀₋₃₀-Carbonsäuren, wobei das Copolymer mit den vorgenannten Vernetzungsagentien vernetzt sein kann. Entsprechende Handelsprodukte sind Pemulen^{®} und die Carbopol^{®}-Typen 954, 980, 1342 und ETD 2020 (ex B. F. Goodrich). Geeignete nichtionische Polymere sind beispielsweise Polyvinylalkohole, die teilverseift sein können, z. B. die Handelsprodukte Mowiol^{®} sowie Vinylpyrrolidon/Vinylester-Copolymere und Polyvinylpyrrolidone, die z. B. unter dem Warenzeichen Luviskol^{®} (BASF) vertrieben werden.

Vorteilhafterweise liegen die erfindungsgemäß verwendeten Hautbehandlungsmittel in Form einer flüssigen oder festen Öl-in-Wasser-Emulsion, Wasser-in-Öl-Emulsion, Mehrfach-Emulsion, Mikroemulsion, PIT-Emulsion oder Pickering-Emulsion, Nanoemulsion, eines Hydrogels, eines Lipogels, einer ein- oder mehrphasigen Lösung, eines Schaumes, eines Puders oder einer Mischung mit mindestens einem als medizinischen Klebstoff geeigneten Polymer vor. Die Mittel können auch in wasserfreier Form, wie beispielsweise einem Öl oder einem Balsam, dargereicht werden. Hierbei kann der Träger ein pflanzliches oder tierisches Öl, ein Mineralöl, ein synthetisches Öl oder eine Mischung solcher Öle sein.

In einer besonderen Ausführungsform der erfindungsgemäß verwendeten Mittel liegen die Mittel als Mikroemulsion vor. Unter Mikroemulsionen werden im Rahmen der Erfindung neben den thermodynamisch stabilen Mikroemulsionen auch die sogenannten "PIT"-Emulsionen verstanden. Bei diesen Emulsionen handelt es sich um Systeme mit den 3 Komponenten Wasser, Öl und Emulgator, die bei Raumtemperatur als Öl-in-Wasser-Emulsion vorliegen. Beim Erwärmen dieser Systeme bilden sich in einem bestimmten Temperaturbereich (als Phaseninversiontemperatur oder "PIT" bezeichnet) Mikroemulsionen aus, die sich bei weiterer Erwärmung in Wasser-in-Öl-Emulsionen umwandeln. Bei anschließendem Abkühlen werden wieder O/W-Emulsionen gebildet, die aber auch bei Raumtemperatur als Mikroemulsionen oder als sehr feinteilige Emulsionen mit einem mittleren Teilchendurchmesser unter 400 nm und insbesondere von etwa 100-300 nm, vorliegen. Erfindungsgemäß können solche Mikro- oder "PIT"-Emulsionen bevorzugt sein, die einen mittleren Teilchendurchmesser von etwa 200 nm aufweisen.

In der Ausführungsform als Emulsion oder als tensidische Lösung, z. B. als Reinigungsmittel, enthalten die erfindungsgemäß verwendeten Zusammensetzungen mindestens eine oberflächenaktive Substanz als Emulgator oder Dispergiermittel. Geeignete Emulgatoren sind beispielsweise Anlagerungsprodukte von 4 bis 30 Mol Ethylenoxid und/oder 0 bis 5 Mol Propylenoxid an lineare C₈-C₂₂-Fettalkohole, an C₁₂-C₂₂-Fettsäuren und an C₈-C₁₅-Alkylphenole, C₁₂-C₂₂-Fettsäuremono- und -diester von Anlagerungsprodukten von 1 bis 30 Mol Ethylenoxid an C₃-C₆-Polyole, insbesondere an Glycerin, Ethylenoxid- und Polyglycerin-Anlagerungsprodukte an Methylglucosid-Fettsäureester, Fettsäurealkanolamide und Fettsäureglucamide, C₈-C₂₂-Alkylmono- und -oligoglycoside und deren ethoxylierte Analoga, wobei Oligomerisierungsgrade von 1,1 bis 5, insbesondere 1,2 bis 2,0, und Glucose als Zuckerkomponente bevorzugt sind, Gemische aus Alkyl-(oligo)-glucosiden und Fettalkoholen, z. B. das im Handel erhältliche Produkt Montanov^{®}68, Anlagerungsprodukte von 5 bis 60 Mol Ethylenoxid an Rizinusöl und gehärtetes Rizinusöl, Partialester von Polyolen mit 3-6 Kohlenstoffatomen mit gesättigten C₈-C₂₂-Fettsäuren, Sterole (Sterine), insbesondere Cholesterol, Lanosterol, Beta-Sitosterol, Stigmasterol, Campesterol und Ergosterol sowie Mykosterole, Phospholipide, vor allem Glucose-Phospolipide, Fettsäureester von Zuckern und Zuckeralkoholen wie Sorbit, Polyglycerine und Polyglycerinderivate, bevorzugt Polyglyceryl-2-dipolyhydroxystearat (Handelsprodukt Dehymuls^{®} PGPH) und Polyglyceryl-3-diisostearat (Handelsprodukt Lameform^{®} TGI) sowie lineare und verzweigte C₈-C₃₀-Fettsäuren und deren Na-, K-, Ammonium-, Ca-, Mg- und Zn - Salze.

Die erfindungsgemäß verwendeten Mittel enthalten die Emulgatoren bevorzugt in Mengen von 0,1 bis 25 Gew.-%, insbesondere 0,5 - 15 Gew.-%, bezogen auf das gesamte Mittel. In einer besonders bevorzugten Ausführungsform ist mindestens ein nichtionischer Emulgator mit einem HLB-Wert von 8 und darunter enthalten. Derart geeignete Emulgatoren sind beispielsweise Verbindungen der allgemeinen Formel R' - O - R², in der R' eine primäre lineare Alkyl-, Alkenyl- oder Acylgruppe mit 20 - 30 C-Atomen und R² Wasserstoff, eine Gruppe mit der Formel -(CₙH₂ₙO)ₓ-H mit x = 1 oder 2 und n = 2 - 4 oder eine Polyhydroxyalkylgruppe mit 4 - 6 C-Atomen und 2 - 5 Hydroxylgruppen ist. Weitere bevorzugt geeignete Emulgatoren mit einem HLB-Wert von 8 und darunter sind die Anlagerungsprodukte von 1 oder 2 Mol Ethylenoxid oder Propylenoxid an Behenylalkohol, Erucylalkohol, Arachidylalkohol oder auch an Behensäure oder Erucasäure. Bevorzugt eignen sich auch die Monoester von C₁₆-C₃₀-Fettsäuren mit Polyolen wie z. B. Pentaerythrit, Trimethylolpropan, Diglycerin, Sorbit, Glucose oder Methylglucose. Beispiele für solche Produkte sind Sorbitan-monobehenat oder Pentaerythrit-monoerucat.

In einer anderen, ebenfalls besonders bevorzugten Ausführungsform ist mindestens ein ionischer Emulgator, ausgewählt aus anionischen, zwitterionischen, ampholytischen und kationischen Emulgatoren, enthalten. Bevorzugte anionische Emulgatoren sind Alkylsulfate, Alkylpolyglycolethersulfate und Ethercarbonsäuren mit 10 bis 18 C-Atomen in der Alkylgruppe und bis zu 12 Glycolethergruppen im Molekül, Sulfobernsteinsäuremono- und -dialkylester mit 8 bis 18 C-Atomen in der Alkylgruppe und Sulfobernsteinsäuremono-alkylpolyoxyethylester mit 8 bis 18 C-Atomen in der Alkylgruppe und 1 bis 6 Oxyethylgruppen, Monoglyceridsulfate, Alkyl- und Alkenyletherphosphate sowie Eiweißfettsäurekondensate. Besonders geeignete zwitterionische Emulgatoren sind die sogenannten Betaine wie die N-Alkyl-N,N-dimethylammonium-glycinate, N-Acyl-aminopropyl--N,N-dimethylammoniumglycinate und 2-Alkyl-3-carboxymethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Beispiele für geeignete ampholytische Emulgatoren sind N-Alkylglycine, N-Alkylaminopropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 24 C-Atomen in der Alkylgruppe. Die ionischen Emulgatoren sind in einer Menge von 0,1 bis 20 Gew.-%, bevorzugt von 1 bis 15 Gew.-% und besonders bevorzugt von 2 bis 10 Gew.-%, bezogen auf das gesamte Mittel, enthalten.

Weitere geeignete Zusatzstoffe sind Fettstoffe, insbesondere pflanzliche Öle, wie Sonnenblumenöl, Olivenöl, Sojaöl, Rapsöl, Mandelöl, Jojobaöl, Orangenöl, Weizenkeimöl, Pfirsichkernöl und die flüssigen Anteile des Kokosöls, flüssige Paraffinöle, Isoparaffinöle und synthetische Kohlenwasserstoffe, Di-n-alkylether mit insgesamt 12 bis 36 C-Atomen, z. B. Di-n-octylether und n-Hexyl-n-octylether, Fettsäuren, besonders lineare und/oder verzweigte, gesättigte und/oder ungesättigte C₈₋₃₀-Fettsäuren, Fettalkohole, besonders gesättigte, ein- oder mehrfach ungesättigte, verzweigte oder unverzweigte Fettalkohole mit 6 - 30 Kohlenstoffatomen, Esteröle, das heißt Ester von C₆₋₃₀-Fettsäuren mit C₂₋₃₀-Fettalkoholen, Hydroxycarbonsäurealkylester, wobei die Vollester der Glycolsäure, Milchsäure, Äpfelsäure, Weinsäure oder Citronensäure bevorzugt sind, Dicarbonsäureester wie Di-n-butyladipat sowie Diolester wie Ethylenglykoldioleat oder Propylenglykoldi(2-ethylhexanoat), symmetrische, unsymmetrische oder cyclische Ester der Kohlensäure mit Fettalkoholen, z. B. Glycerincarbonat oder Dicaprylylcarbonat (Cetiol^{®} CC), Mono,- Di- und Trifettsäureester von gesättigten und/oder ungesättigten linearen und/oder verzweigten Fettsäuren mit Glycerin Wachse, insbesondere Insektenwachse, Pflanzenwachse, Fruchtwachse, Ozokerit, Mikrowachse, Ceresin, Paraffinwachse, Triglyceride gesättigter und gegebenenfalls hydroxylierter C₁₆₋₃₀-Fettsäuren, z. B. gehärtete Triglyceridfette, Siliconverbindungen, ausgewählt aus Decamethylcyclopentasiloxan, Dodecamethylcyclohexasiloxan und Siliconpolymeren, die gewünschtenfalls quervernetzt sein können, z. B. Polydialkylsiloxane, Polyalkylarylsiloxane, ethoxylierte Polydialkylsiloxane sowie Polydialkylsiloxane, die Amin- und/oder Hydroxy-Gruppen enthalten.

Die Einsatzmenge der Fettstoffe beträgt 0,1 - 50 Gew.%, bevorzugt 0,1 - 20 Gew.% und besonders bevorzugt 0,1 - 15 Gew.%, jeweils bezogen auf das gesamte Mittel.

Die erfindungsgemäß verwendeten Mittel können weitere Wirk-, Hilfs- und Zusatzstoffe enthalten, beispielsweise Vitamine, Provitamine und Vitaminvorstufen aus den Gruppen A, B, C, E und F, Allantoin, Bisabolol, Antioxidantien, zum Beispiel Imidazole (z. B. Urocaninsäure) und deren Derivate, Chlorogensäure und deren Derivate, Liponsäure und deren Derivate (z. B. Dihydroliponsäure), Aurothioglucose, Propylthiouracil und andere Thiole (z. B. Thioredoxin, Glutathion, Cystein, Cystin, Cystamin und deren Glycosyl-, N-Acetyl-, Methyl-, Ethyl-, Amyl-, Propyl-, Butyl-, Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z. B. Buthioninsulfoximine, Homocysteinsulfoximin, Butioninsulfone, Penta-, Hexa-, Heptathioninsulfoximin) in sehr geringen verträglichen Dosierungen (z. B. pmol bis µmol/kg), ferner (Metall)-Chelatoren (z. B. Phytinsäure, Lactoferrin), Huminsäuren, Gallensäuren, Gallenextrakte, Bilirubin, Biliverdin, Ubichinon und Ubichinol und deren Derivate, das Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Harnsäure und deren Derivate, Katalase, Superoxid-Dismutase, Zink und dessen Derivate (z. B. ZnO, ZnSO₄), Selen und dessen Derivate (z. B. Selen-Methionin), Stilbene und deren Derivate (z. B. Stilbenoxid, trans-Stilbenoxid) und die als Antioxidans geeigneten Derivate (Salze, Ester, Ether, Zucker, Nukleotide, Nukleoside, Peptide und Lipide) dieser Wirkstoffe, weiterhin Ceramide und Pseudoceramide, Triterpene, insbesondere Triterpensäuren wie Ursolsäure, Rosmarinsäure, Betulinsäure, Boswelliasäure und Bryonolsäure, monomere Catechine, besonders Catechin und Epicatechin, Leukoanthocyanidine, Catechinpolymere (Catechin-Gerbstoffe) sowie Gallotannine, Verdickungsmittel, z. B. natürliche und synthetische Tone und Schichtsilikate wie Bentonit, Hectorit, Montmorillonit oder Laponite^{®}, Dimethylisosorbid, Alpha-, Beta- sowie Gamma-Cyclodextrine, Lösungsmittel, Quell- und Penetrationsstoffe wie Ethanol, Isopropanol, Ethylenglykol, Propylenglykol, Propylenglykolmonoethylether, Glycerin und Diethylenglykol, Carbonate, Hydrogencarbonate, Guanidine, Harnstoffe sowie primäre, sekundäre und tertiäre Phosphate, Parfümöle, Pigmente sowie Farbstoffe zum Anfärben des Mittels, Substanzen zur Einstellung des pH-Wertes, Komplexbildner wie EDTA, NTA, β-Alanindiessigsäure und Phosphonsäuren, Perlglanzmittel wie Ethylenglykolmono- und -distearat, Trübungsmittel wie Latex, Styrol/PVP- und Styrol/Acrylamid-Copolymere und Treibmittel wie Propan-Butan-Gemische, N₂O, Dimethylether, CO₂ und Luft.

Die folgenden Beispiele sollen die vorliegende Erfindung verdeutlichen, ohne sie hierauf zu beschränken.

### Experimenteller Teil

### 1. Untersuchungen an Mehrschicht-Hautmodellen

Die Wirkung von Ederline L wurde an einem mehrschichtigen in-vitro-Hautmodell untersucht. Das Hautmodell ist ein humanes Hautäquivalent, das aus einer Dermis mit Fibroblasten und einer Epidermis aus Keratinozyten besteht.

Diese Mehrschicht-Struktur entsteht in einem speziellen Kultivierungsverfahren. Es wurden zunächst dermale Äquivalente (DE) produziert, indem eine Suspension von 2 x 10⁵/cm² Fibroblasten aus humaner Vorhaut in einem Kulturmedium auf eine aus Chitosan, Collagen und Glycosaminoglycanen bestehende Matrix aufpipettiert wurde (Matrix beschrieben bei Collombel, C. et al.: Biomaterials with a base of collagen, chitosane and glycosaminoglycans, process for preparing them and their application in human medicine, US Patent 5166187). Das Kulturmedium bestand aus Dulbecco's Modified Eagle's Medium (DMEM), supplementiert mit 10% fetalem Kälberserum (FCS), 25 µg/ml Gentamycin, 100 UI/ml Penicillin, 1 µg/ml Amphotericin B, 50 µg/ml Natriumascorbat und 4 mM L-Glutamin. Die dermalen Äquivalente wurden 14 Tage in diesem Medium bei 37°C in einer Atmosphäre CO₂/Luft (5%/95%, v/v) und 90% Luftfeuchtigkeit inkubiert, wobei das Medium jeden Tag erneuert wurde. Für die Hautäquivalente (SE) wurden Keratinozyten aus humaner Vorhaut in einer Dichte von 200.000 Zellen/cm² auf die 14 Tage alten DEs ausgesät und unter submersen Bedingungen in einem Medium, bestehend aus 60 % DMEM, 30 % HAM F12 und 10% FCS, supplementiert mit 25 µg/ml Gentamycin, 100 UI/ml Penicillin, 1 µg/ml Amphotericin B, 50 µg/ml Natriumascorbat, 4 mM L-Glutamin, 10 ng/ml Epidermalem Wachstumsfaktor (EGF), 0,4µg/ml Hydrocortison, 0,12UI/ml Insulin, 10⁻⁹ M Choleratoxin, 5 ng/ml Transferrin und 180 µM Adenin, für weitere 7 Tage inkubiert. Die Hautäquivalente wurden dann an der Luft-Flüssigkeitsgrenze (Air-Liquid-Interface) für weitere 14 Tage in modifiziertem Keratinozytenmedium (DMEM-HAM F12, supplementiert mit 0,4 µg/ml Hydrocortison und 0,12 UI/ml Insulin) kultiviert.

Im Vergleich zu üblicherweise verwendeten Monolayer-Kulturen entspricht dieses Ganzhautmodell sehr viel besser der in-vivo-Situation, da Keratinozyten und Fibroblasten in engem Kontakt zueinander stehen und, wie in vivo, Signalstoffe austauschen können.

### 2. Behandlung der Ganzhautmodelle:

Zur Durchführung der Prüfungen wurden Cremeformulierungen auf Basis der nachstehend aufgeführten Versuchsrezeptur mit 3 Gew.-% und mit 5 Gew.-% Ederline L hergestellt, wobei die Differenz zu 100 Gew.-% mit Wasser ausgeglichen wurde. Anschließend wurden die Effekte der Apfelkernextrakt-haltigen Cremes auf Ganzhautmodelle im Vergleich zu einer Placebocreme (gleiche Cremeformulierung ohne Ederline L) bewertet.

Dazu wurden die Hautmodelle topisch mit jeweils 5µl der verschiedenen Cremeformulierungen behandelt, anschließend über 6 Stunden bzw. 48 Stunden inkubiert und die Genexpression der mit Creme behandelten Hautmodelle im Vergleich mit der Genexpression unbehandelter Hautmodelle ermittelt.

In einem zweiten Testansatz wurden Ganzhautmodelle über 9 Tage insgesamt viermal mit den genannten Cremeformulierungen behandelt und Effekte auf histologischer Ebene und der Proteinebene ermittelt.

Cremeformulierung der Versuchsrezepturen (Angaben in Gew.-%)

| | |
|---|---|
| Montanov^{®} 68 | 6,00 |
| Myritol^{®} 318 | 7,00 |
| Stenol^{®} 16/18 | 1,25 |
| Cutina^{®} MDV | 2,50 |
| Novata^{®} AB | 3,00 |
| Cetiol^{®} SB 45 | 1,50 |
| Eusolex^{®} 4360 | 0,50 |
| Tocopherylacetat | 0,50 |
| Baysilon^{®} M350 | 0,50 |
| PHB-Propylester | 0,20 |
| Generol^{®} R | 0,50 |
| TiO₂ | 0,30 |
| Tego Carbomer 2%ig | 10,00 |
| Talkum DAB | 0,50 |
| Glycerin | 4,50 |
| Sorbit 70%-ig | 2,00 |
| p-Hydroxybenzoesäuremethylester | 0,20 |
| NaOH 10%-ig | pH 4,8-5,2 |
| Wasser, dest | ad 100 |

### 3. RNA-Präparation aus den Ganzhautmodellen:

Die RNA-Präparationen wurden 6 Stunden und 48 Stunden nach der Cremeapplikation durchgeführt.

Vor der Isolation der RNA (Ribonucleinsäure) wurden die Hautmodelle unter Ribonuclease-freien Bedingungen vom Filterpapier abgenommen, in einem Gefäß mit flüssigem Stickstoff eingefroren und anschließend bis zur Aufarbeitung in flüssigem Stickstoff gelagert.

Zur RNA-Präparation wurden die Modelle gemäß eines modifizierten Protokolls der Firma Qiagen (RNeasy Protocol: Isolation of total RNA from Heart, Muscle and Skin Tissue; TS-RY7; 05/99) aufgearbeitet. Die RNA wurde photometrisch vermessen. Die RNA-Ausbeute (siehe Tabelle 1) gibt bereits Hinweise auf die Wirksamkeit einer Substanz.

**Tabelle 1: RNA-Gesamtausbeute behandelter und unbehandelter Ganzhautmodelle nach 6 Stunden und 48 Stunden [in µg RNA]**

| | **unbehandelt** | **mit Placebo-Cre me behandelt** | **mit Creme mit 3 Gew.-% Ederline L behandelt** | **mit Creme mit 5 Gew.-% Ederline L behandelt** |
|---|---|---|---|---|
| **nach 6 Stunden** | 34 | 27 | 42 | 79 |
| **nach 48 Stunden** | 32 | 24 | 47 | 47 |

Gegenüber den unbehandelten beziehungsweise mit Placebo-Creme behandelten Ganzhautmodellen zeigte sich bei der Behandlung mit Apfelkernextrakt-haltigen Cremes eine deutliche Steigerung der RNA-Ausbeute.

### 4. Genexpressionsanalysen

Die Genexpressionsanalysen wurden mittels cDNA-Arrays (Arrays mit komplementärer DNA) durchgeführt. Für die vorliegende Studie wurden cDNA-Arrays verwendet, die unterschiedliche cDNAs der Spezies Mensch tragen und alle in humaner Haut aktiv sind. Als Positivkontrolle wurden zusätzlich "house keeping"-Gene und E. coli-DNA-Fragmente aufgetragen. Als Negativkontrollen wurden Heringssperma-DNA und Puffer mit aufgetragen.

Die PCR-Amplifikate der klonierten cDNA-Fragmente wurden auf eine einheitliche Konzentration eingestellt und mit einem Dispensiergerät auf die Oberfläche derivatisierter Objektträger aufgetropft. Für die Bestimmung der Genexpressionsprofile wurde jeweils RNA eines behandelten Hautmodells mit dem Fluoreszenzfarbstoff Cy5 und die RNA des entsprechend unbehandelten Hautmodells (Kontrolle) mit dem Fluoreszenzfarbstoff Cy3 markiert und gemeinsam auf einem cDNA-Array hybridisiert. Die Markierung wurde durch eine reverse Transkription unter Einbau von fluoreszenzmarkierten Nukleotiden (Cy3-dCTP bzw. Cy5-dCTP) erreicht. Die hybridisierten Arrays wurden mit einem Laser Scanning Gerät ausgelesen. Die jeweils für beide Fluoreszenzmarkierungen erhaltenen Bilder werden für die weitergehende Analyse digital übereinander gelegt ("overlay"). Hierbei bedeutet die Farbe Grün, daß die Cy5 Fluoreszenz eine höhere Intensität als Cy3 hat, Rot, daß die Cy3 Fluoreszenz eine höhere Intensität als Cy5 hat, Gelb, daß beide Fluoreszenzen die gleiche Intensität aufweisen und damit auch das entsprechende Gen in beiden Proben gleich stark exprimiert wurde.

Zur Erstellung der Expressionsprofile wurden zunächst die Cy3 und Cy5 Signal- und Backgroundintensitäten der hybridisierten Arrays bestimmt. Die Background-Werte wurden von den Signalintensitäten subtrahiert, es wurde der Mittelwert der Doppelspots berechnet und abschließend der Quotient aus Cy5/Cy3-Signal berechnet. Die Werte wurden über den Median aller Signalquotienten normiert.

Für die weitere Auswertung wurden nur solche Signalpaare (Cy3 und Cy5) verwendet, bei denen nach Backgroundsubtraktion zumindest eine der beiden Signalintensitäten mindestens 3fach über der Signalintensität der Negativkontrollen (Heringssperma-DNA und Puffer) lag. Dadurch war gewährleistet, dass sehr schwache Signalintensitäten, die äußerst sensitiv auf einen leicht variierenden Background oder eine unspezifische Hybridisierung reagieren, ausgeschlossen wurden.

Für die Bewertung des Apfelkernextraktes als Wirkstoff war es von Interesse herauszufinden, welche Gene im Ganzhautmodell durch Zugabe des Apfelkernextraktes reguliert sind. Dabei waren Effekte, die auf einer mindestens zweifach differentiellen Expression beruhen, signifikant und wurden weiter ausgewertet.

**Tabelle 2: Relative Genexpression in mit 5 Gew.-% Ederline L-haltiger Creme behandelten Hautmodellen nach 6 Stunden und nach 48 Stunden, bezogen auf unbehandelte Haut und verglichen mit der Genexpression in gealterter Haut (69 Jahre) im Verhältnis zu junger Haut (29 Jahre)**

| | **Expression in gealterter Haut zu Expression in junger Haut** | **Expression bei Creme-behandlung mit 5 Gew.-% Ederline L nach 6 h zu Expression in unbehan-delter Haut** | **Expression bei Creme-behandlung mit 5 Gew.-% Ederline L nach 48 h zu Expression in unbehan-delter Haut** |
|---|---|---|---|
| **PGS 2** | +1,5 | -1,7 | -2,1 |
| **CD 44** | -2,1 | +2,2 | +2,4 |
| **GJB 2** | -2,1 | +2,9 | +2,1 |
| **PSMD 2** | -2,1 | +2,5 | +1,6 |
| **DBPA** | -3,3 | +2,2 | +2,2 |
| **FAS** | -3,3 | +3,0 | +2,3 |

Tabelle 2 ist beispielsweise zu entnehmen, dass in gealterter Haut (69 Jahre) im Verhältnis zu junger Haut (29 Jahre) das Gen PGS 2 um den Faktor 1,5 stärker exprimiert ist. Die übrigen aufgelisteten Gene sind in der gealterten Haut um den Faktor 2,1 bzw. 3,3 schwächer exprimiert. Die Genexpression in mit Apfelkernextrakt-Creme behandelten Hautmodellen gegenüber der Genexpression in unbehandelten Hautmodellen zeigt für jedes untersuchte Gen einen gegenläufigen Trend. Somit verändert die Behandlung mit Apfelkernextrakten ein Genexpressionsprofil, bestehend aus sechs Altersmarkem der Haut, in die Richtung eines Genexpressionsprofils von jüngerer Haut.

**Tabelle 3: Aktivierung der Gene für Connexin 43 und Connexin 26 durch Behandlung mit Apfelkernextrakt im Verhältnis zum Placebo-behandelten Hautmodell**

| | **Expression bei Placebo-Behandlung zu Expression in unbehan-delter Haut** | **Expression bei Creme-behandlung mit 5 Gew.-% Ederline L nach 6 h zu Expression in unbe-handelter Haut** | **Expression bei Creme-behandlung mit 5 Gew.-% Ederline L nach 48 h zu Expression in unbehandelter Haut** |
|---|---|---|---|
| **Connexin 43** | 1,5 | 2,6 | 2,3 |
| **Connexin 26** | 1,5 | 2,9 | 2,1 |

Die Behandlung von Hautmodellen mit Apfelkernextrakten resultierte in einer gesteigerten Expression der Gene für Connexin 43 und Connexin 26. Die Werte oberhalb von 2 sind statistisch signifikant, da sie die biologische Schwankung mit berücksichtigen.

**Tabelle 4: Aktivierung der Gene für die Proteine Keratin 10, Keratin 1, Keratin 5, Keratin 14 und den Hyaluronat-Rezeptor CD 44 durch Behandlung mit Apfelkernextrakt im Verhältnis zum Placebo-behandelten Hautmodell**

| | **Expression bei Placebo-Behandlung zu Expression in unbehandelter Haut** | **Expression bei Cremebehandlung mit 5 Gew.-% Ederline L nach 6 h zu Expression in unbehandelter Haut** | **Expression bei Cremebehandlung mit 5 Gew.-% Ederline L nach 48 h zu Expression in unbehandelter Haut** |
|---|---|---|---|
| **KRT 10** | 1,3 | 3,4 | 2,0 |
| **KRT 1** | 1,0 | 3,5 | 2,0 |
| **KRT 5** | 1,1 | 2,3 | 1,5 |
| **KRT 14** | 1,0 | 2,4 | 1,3 |
| **CD 44** | 1,5 | 2,2 | 2,4 |

Die Behandlung von Hautmodellen mit Apfelkernextrakten resultierte in einer gesteigerten Expression der Gene für verschiedene Keratine und den Hyaluronat-Oberflächenrezeptor CD 44. Somit haben die CD44-exprimierenden Zellen eine verstärkte Kapazität, an extrazelluläre Hyaluronsäure zu binden. Die Werte oberhalb von 2 sind statistisch signifikant, da sie die biologische Schwankung mit berücksichtigen.

**Tabelle 5: Aktivierung der Gene für Fatty Acid Synthase (FAS) und Fatty Acid Binding Protein in Epidermis (FABE) durch Behandlung mit Apfelkernextrakt im Verhältnis zum Placebo-behandelten Hautmodell**

| | **Expression bei Placebo-Behandlung nach 48 h zu Expression in unbehandelter Haut** | **Expression bei Creme-behandlung mit 5 Gew.-% Ederline L nach 6h zu Expression in unbehan-delter Haut** | **Expression bei Creme-behandlung mit 5 Gew.-% Ederline L nach 48 h zu Expression in unbehandelter Haut** |
|---|---|---|---|
| **FAS** | 1,4 | 3,0 | 2,3 |
| **FABE** | 1,5 | 2,4 | 1,7 |

Die Behandlung von Ganzhautmodellen mit Apfelkernextrakt-haltigen Cremes führte zu einer gesteigerten Expression der Gene für FAS und FABE. Es ist anzunehmen, dass sich dieser Effekt auch auf der Ebene der Proteine und deren Aktivität auswirkt, so dass davon ausgegangen werden kann, dass der Lipidgehalt der Haut durch eine gesteigerte Lipidsynthese in der Epidermis erhöht und somit die Barrierefunktion verbessert wird.

### 5. Epidermisdicke

Die Behandlungszeit vor Messung der Epidermisdicke betrug 9 Tage.

Nach Ende der Behandlungszeit wurden die Hautmodelle in OCT-Medium eingebettet und mittels eines Kryostaten Schnitte mit einer Dicke von jeweils 4µm angefertigt.

Zur Kontrolle der Epidermisdicke wurden die Präparate mit Hämatoxilin/Eosin, einer in der dermatologischen Histologie üblichen Färbetechnik, gefärbt (H&E-Färbung) und die Schichtdicke der lebenden Zellschichten (Epidermis ohne Stratum corneum) an jeweils 3 verschiedenen Schnitten pro Hautmodell (bei n = 3 Parallelkulturen) an 3 repräsentativen Stellen mittels Bild-Analyse vermessen.

Nach topischer Behandlung über 9 Tage erschien die histologische Struktur des Ganzhautmodells und die Ausbildung der Zellschichten bei allen 3 Behandlungsarten regelrecht. Die lebenden Schichten der Epidermis waren nach Behandlung mit Apfelkernextrakt-haltigen Cremes im Vergleich zur Placebo-Behandlung verdickt. Mit zunehmender Ederline L-Konzentration beobachtete man eine Zunahme der Dicke des Stratum corneums.

**Tabelle 6: Dicke der lebenden Epidermisschichten (ohne Stratum corneum) nach topischer Behandlung mit Placebocreme, Creme mit 3 Gew.-% Ederline L und Creme mit 5 Gew.-% Ederline L viermalig über insgesamt 9 Tage.**

| | **Placebo-Behandlung** | **Cremebehandlung mit 3 Gew.-% Ederline L** | **Cremebehandlung mit 5 Gew.-% Ederline L** |
|---|---|---|---|
| **Relative Schichtdicke** | 100 +/- 5 % | 125 +/- 3 % | 115 +/- 8 |

Die lebenden Schichten der Epidermis sind im Vergleich zur Placebo-Behandlung verdickt, und zwar um ca. 25% bei Behandlung mit der Creme mit 3 Gew.-% Ederline L und um ca. 15% bei Behandlung mit der Creme mit 5 Gew.-% Ederline L.

### 6. Hyaluronsäuregehalt

Der Hyaluronsäuregehalt wurde aus dem Medium mit Hilfe des "Hyaluronic Acid"-Kits (HA-Assay, Akagi Trading Co. Ltd., Kobe Hyogo, Japan) nach Herstellerangaben ermittelt. Die Ganzhautmodelle wurden an den Versuchstagen 0, 2, 4 und 7 mit den jeweiligen Cremes behandelt. Die Probennahme für die Analyse des Hyaluronsäuregehalts erfolgte an den Versuchstagen 1, 3 und 8, das heißt, jeweils einen Tag nach erfolgter Creme-Behandlung. Die Behandlung erfolgte also insgesamt viermal innerhalb eines Zeitraumes von 9 Tagen.

**Tabelle 7: Hyaluronsäure-Gehalt im Medium von Ganzhautmodellen 1, 3 und 8 Tage nach erstmaliger topischer Behandlung mit Placebocreme und Creme mit 5 Gew.-% Ederline L (in µg Hyaluronsäure pro ml Medium].**

| | **unbehandelt** | **mit Placebo-Cre me behandelt** | **mit Creme mit 5 Gew.-% Ederline L behandelt** |
|---|---|---|---|
| **Versuchstag 1** | 818 | 1045 | 825 |
| **Versuchstag 3** | 1182 | 1318 | 1409 |
| **Versuchstag 8** | 1364 | 1591 | 1909 |

Die Behandlung mit der 5 Gew.-% Ederline L enthaltenden Creme führte zu einer Steigerung der Hyaluronsäure-Synthese, insbesondere nach 8tägiger Behandlungsdauer.

**Tabelle 8: Zusammenstellung der untersuchten Gene**

| **Nr.** | **Unigene Accession No(s)** | **Swiss-Prot-Numm er** | **Beschreibung** |
|---|---|---|---|
| 1 | Hs.195850 | P13647 | KRT5: KERATIN, TYPE II CYTOSKELETAL 5 (CYTOKERATIN 5) (K5) (CK 5) (58 KDA CYTOKERATIN) (epidermolysis bullosa simplex, Dowling-Meara/Kobner/Weber-Cockayne types) |
| 2 | Hs.117729 | P02533 | Keratin 14 |
| 3 | Hs.99936 | P13645 | KRT10: KERATIN, TYPE I CYTOSKELETAL 10 (CYTOKERATIN 10) (K10) (epidermolytic hyperkeratosis; keratosis palmaris et plantaris) |
| 4. | Hs.80828 | P04264 | Keratin 1 |
| 5 | Hs.83190 | P49327 | FAS: FATTY ACID SYNTHASE (EC 2.3.1.85) [INCLUDES: EC 2.3.1.38; EC 2.3.1.39; EC 2.3.1.41; EC 1.1.1.100; EC 4.2.1.61; EC 1.3.1.10; EC 3.1.2.14). |
| 6 | Hs.153179 | Q01469 | FABE: FATTY ACID BINDING PROTEIN, EPIDERMAL (E FABP) (PSORIASIS ASSOCIATED FATTY ACID BINDING PROTEIN HOMOLOG) (PA FABP)(FABPS) |
| 7 | Hs.1139 | P16989 | DBPA: HUMAN (CSDA OR DBPA) DNA |
| | | | BINDING PROTEIN A (COLD SHOCK DOMAIN |
| 8 | Hs.74619 | Q13200 | PSMD2: (PSMD2 OR TRAP2) 26S PROTEASOME REGULATORY SUBUNIT S2 (P97) (TUMOR NECROSIS FACTOR TYPE 1 RECEPTOR ASSOCIATED PROTEIN 2). |
| 9 | Hs.169610 | P16070 | CD44 |
| 10 | | P29033 | CXB2: GAP JUNCTION BETA 2 PROTEIN 26kD (CONNEXIN 26) (CX26) |
| 11 | Hs.74471 | P17302 | gap junction alpha 1 protein, 43kD (connexin 43) |
| 12 | Hs.76152 | P07585 | PGS2: BONE PROTEOGLYCAN II PRECURSOR (PG S2) (DECORIN) (PG40) |

### Weitere Rezepturbeispiele

### Beispiel 1: Cremeformulierung (Angaben in Gew.-%)

| | |
|---|---|
| Isopropylpalmitat | 5,00 |
| Cutina^{®} MDV | 2,00 |
| Stenol^{®} 1618 | 1,00 |
| Baysilon^{®} M 350 | 0,50 |
| Biophilic^{®} H | 4,00 |
| 1,6-Hexandiol | 6,00 |
| Glycerin | 5,00 |
| Trilon^{®} A | 0,10 |
| Ederline^{®} L | 3,00 |
| Tego Carbomer, 2%ig | 20,00 |
| Wasser | ad 100 |

### Beispiel 2: Cremeformulierung (Angaben in Gew.-%)

| | |
|---|---|
| Emuliance^{®} | 4,00 |
| Myritol^{®} 318 | 6,00 |
| Cutina^{®} MDV | 2,00 |
| Stenol^{®} 1618 | 1,00 |
| Baysilon^{®} M 350 | 0,50 |
| 1,6-Hexandiol | 6,00 |
| Glycerin | 5,00 |
| Ederline L | 3,00 |
| Wasser | ad 100 |

### Beispiel 3: Cremeformulierung (Angaben in Gew.-%)

| | |
|---|---|
| Montanov^{®} 202 | 3,00 |
| Isopropylstearat | 3,00 |
| Myritol^{®} 331 | 1,00 |
| Performalene^{®} 400 | 1,00 |
| Cegesoft^{®} C 24 | 3,00 |
| Lanette^{®} 22 | 1,00 |
| Cutina^{®} MDV | 2,00 |
| Tocopherylacetat | 0,50 |
| Controx^{®} KS | 0,25 |
| Parsol^{®} 1789 | 1,00 |
| Eusolex^{®} 6300 | 2,00 |
| Uvinul^{®} T 150 | 1,25 |
| Baysilon^{®} M350 | 0,50 |
| Tego Carbomer 140 2 %ig | 25,00 |
| 1,6 - Hexandiol | 6,00 |
| Glycerin | 5,00 |
| 1,2-Propylenglykol | 5,00 |
| DSH-CN | 2,00 |
| NaOH 10 %ig | ph 4,8-5,2 |
| Dry Flo Plus | 1,00 |
| Ederline L | 3,00 |
| Wasser demin. | ad 100 |

### Beispiel 4: Cremeformulierung (Angaben in Gew.-%)

| | |
|---|---|
| Distelöl | 3,0 |
| Myritol^{®} PC | 3,5 |
| Lanette^{®} 22 | 3,0 |
| Cutina^{®} GMS-V | 3,0 |
| Stenol^{®} 16/18 | 2,0 |
| Isopropylstearat | 6,0 |
| Baysilon^{®} M350 | 1,0 |
| Controx^{®} KS | 0,05 |
| p-Hydroxybenzoesäurepropylester | 0,2 |
| Glycerin | 5,0 |
| p-Hydroxybenzoesäuremethylester | 0,2 |
| Hibiscin^{®} HP LS 9198 | 3,0 |
| TiO₂ | 0,5 |
| Citronensäure | 0,1 |
| Ederline L | 3,0 |
| Calciumpantothenat | 0,048 |
| Sepigel^{®} 305 | 2,0 |
| Wasser | ad 100 |

### Beispiel 5 Cremeformulierung (Angaben in Gew.-%)

| | |
|---|---|
| Lipoid S 75-3 | 1,50 |
| Baysilon^{®} M 350 | 1,00 |
| Cetiol^{®} J 600 DEO | 4,00 |
| Cetiol^{®} SB 45 | 3,00 |
| Stenol^{®} 1618 gesch. | 0,50 |
| Cutina^{®} MD-V | 1,00 |
| Floraesters 70 | 1,50 |
| Controx^{®} KS | 0,20 |
| Mandelöl | 2,00 |
| p-Hydroxybenzoesäurepropylester | 0,20 |
| Tego Carbomer 140 2 %ig | 20,00 |
| Talkum Pharma G | 1,00 |
| Glycerin | 3,00 |
| Dipropylenglycol | 6,00 |
| p-Hydroxybenzoesäuremethylester | 0,20 |
| Dry Flo PLUS | 1,00 |
| Retinylpalmitat | 0,10 |
| DSH-CN | 2,00 |
| Ederline L | 3,00 |
| NaOH 10 %ig | 1,50 |
| Wasser | ad 100 |

### Beispiel 6 Cremeformulierung (Angaben in Gew.-%)

| | |
|---|---|
| Baysilon^{®} M 350 | 1,00 |
| Cetiol^{®} OE | 5,00 |
| Cegesoft C 24 | 5,00 |
| Stenol^{®} 1618 gesch. | 2,00 |
| Cutina^{®} MD-V | 1,00 |
| Tego Care CG 90 | 1,00 |
| p-Hydroxybenzoesäurepropylester | 0,10 |
| Glycerin | 5,00 |
| Sorbitol | 3,00 |
| Glucose | 1,00 |
| p-Hydroxybenzoesäuremethylester | 0,10 |
| Ederline L | 5,00 |
| Wasser | ad 100 |

### Beispiel 7 Reinigungsmilch (Angaben in Gew.-%)

| | |
|---|---|
| Tego Carbomer 140 2 %ig | 20,00 |
| Benecel | 0,30 |
| Paraffinöl | 20,00 |
| Stenol^{®} 1618 gesch. | 2,00 |
| Hostaphat KW 340 D | 3,00 |
| Eumulgin B1 | 1,50 |
| Tocopherylacetat | 0,50 |
| p-Hydroxybenzoesäurepropylester | 0,20 |
| Glycerin | 5,00 |
| Hexandiol | 3,00 |
| p-Hydroxybenzoesäuremethylester | 0,20 |
| Ederline L | 5,00 |
| Glucono-Δ-lacton | 2,00 |
| Phenoxyethanol | 0,40 |
| Triton M | 0,10 |
| Wasser | ad 100 |

### Beispiel 8 Leave-on Haarkur (Angaben in Gew.-%)

| | |
|---|---|
| MONOMULS^{®} 60-35 C | 1,24 |
| EUMULGIN^{®} B1 | 2,76 |
| Cetiol S | 9,00 |
| Cetiol OE | 9,00 |
| Dow Corning DC 345^{®} | 2,00 |
| GLUADIN^{®} WQ | 2,85 |
| PLANTACARE^{®} 2000 UP | 2,00 |
| Ederline L | 5,00 |
| Wasser | ad 100 |

### Liste der verwendeten Inhaltsstoffe

| **Komponente** | **INCI-Bezeichung** | **Hersteller** |
|---|---|---|
| Baysilonöl^{®} M350 | Dimethicone | GE-Bayer-Silicones |
| Benecel MP 333 C | Hydroxypropyl Methylcellulose | Hercules |
| Biophilic H | Hydriertes Lecithin, Fettsäuren, Fettalkohole | Lucas Meyer |
| Cegesoft C 24 | Ethylhexyl Palmitate | Cognis |
| Cetiol^{®} J 600 DEO | Oleoyl Erucate | Cognis |
| Cetiol^{®} OE | Dicaprylyl Ether | Cognis |
| Cetiol^{®} S | Dioctylcyclohexane | Cognis |
| Cetiol^{®} SB 45 | Butyrospermum Parkii (Linne) | Cognis |
| Controx^{®} KS: | Tocopherol, Hydrogenated Palm Glycerides Citrate | Cognis |
| Cutina^{®} GMS (C 16-18-Fettsäure-mono-diglycerid | Glyceryl Stearate | Cognis |
| Cutina^{®} MDV (C 16-18-Fettsäure-mono-diglycerid | Glyceryl Stearate | Cognis |
| Dow Corning DC 345 | Cyclomethicone | Dow Corning |
| Dry Flo Plus | Aluminium Starch Octenylsuccinate | National Starch and Chemical Company |
| DSH-C-N | Dimethylsilanol Hyaluronate (wässrige Lösung) | Exsymol |
| Emuliance | Cetearyl Wheat Bran Glycosides, Cetearyl Alcohol | Soliance |
| Eumulgin B1 | Ceteareth-12 | Cognis |
| Eusolex^{®} 4360 | Benzophenone-3 | Merck |
| Eusolex^{®} 6300 | 4-Methylbenzylidene Camphor | Merck |
| Floraesters 60 | Jojoba Esters | Flora Technologies |
| Floraesters 70 | Jojoba Esters | Flora Technologies |
| Generol^{®} R | Brassica Campestris (Rapeseed) Sterols | Cognis |
| Gluadin^{®} WQ | Laurdimonium Hydroxypropyl Hydrolyzed Wheat Protein (31 %) | Cognis |
| Hibiscin^{®} HP-LS-9198 | Water, Hibiscus esculentus Seed Extract, Phenoxyethanol | Laboratoires Sérobiologiques |
| Hostaphat KW 340 D | C 16-18-Fettalkohol+4-EO-ortho-Ph osphorsäure-mono-di-tri-ester | Clariant |
| Lanette^{®} 22 | Behenyl Alcohol | Cognis |
| Lipoid S 75-3 | Hydrogenated Lecithin | Lipoid GmbH |
| MONOMlJLS^{®} 60-35 C | Hydrogenated Palm Glycerides | Cognis |
| Montanov^{®} 202 | Arachidyl Alcohol, Behenyl Alcohol, Arachidyl Glucoside | SEPPIC |
| Myritol^{®} 318 | Caprylic/Capric Triglyceride | Cognis |
| Myritol^{®} 331 | Cocoglycerides | Cognis |
| Myritol^{®} PC | Propylene Glycol Dicaprylate/Dicaprate | Cognis |
| Novata^{®} AB | Cocoglycerides | Cognis |
| Parsol^{®} 1789 | Butyl Methoxydibenzoylmethane | Roche |
| Performalene 400-Polyethylene | Polyethylene | New Phase Technologies |
| PLANTACARE^{®} 2000 UP | Decyl Glucoside (ca. 50 %) | Cognis |
| Sepigel^{®} 305 | Polyacrylamide, C13-14 Isoparaffin, Laureth-7 | SEPPIC |
| Stenol^{®} 16/18 | Cetearyl Alcohol | Cognis |
| Tego Care CG 90 | Cetearyl Glucoside, mind. 90 % Aktivsubstanz | Goldschmidt |
| Trilon^{®} A | Nitrilotriessigsäure 3 Na | BASF |
| Trilon^{®} M | Methylglycindiessigsäure-tri-Natriu m-Salz | BASF |
| Uvinul^{®} T 150 | Octyl Triazone | BASF |

## Patentansprüche

1. Nicht-therapeutische, kosmetische topische Verwendung von Apfelkernextrakten zur Steigerung der interzellulären Zellhaftung in der Haut und den Hautanhangsgebilden.

2. Nicht-therapeutische, kosmetische topische Verwendung von Apfelkernextrakten zur Steigerung der Haftung zwischen Zellen und der extrazellulären Matrix in der Haut und den Hautanhangsgebilden.

3. Nicht-therapeutische, kosmetische topische Verwendung von Apfelkernextrakten zur Steigerung der Lipidproduktion in der Epidermis.

4. Nicht-therapeutische, kosmetische topische Verwendung von Apfelkernextrakten zur Steigerung der Ausbildung von interzellulären Kommunikationsporen in der Haut und den Hautanhangsgebilden und zur Verbesserung der interzellulären Zellkommunikation.

5. Nicht-therapeutische, kosmetische topische Verwendung von Apfelkernextrakten zur Steigerung der Expression
- des Keratins 5 (KRT5) mit der Swiss-Prot-Nummer P13647,
- des Keratins 14 (KRT14) mit der Swiss-Prot-Nummer P02533,
- des Keratins 10 (KRT10) mit der Swiss-Prot-Nummer P 13645,
- des Keratins 1 (KRT1) mit der Swiss-Prot-Nummer P04264,
- des Gap Junction Proteins Beta 2 Connexin 26 (CXB2) mit der Swiss-Prot-Nummer P29033,
- des Gap Junction Proteins Alpha 1 Connexin 43 mit der Swiss-Prot-Nummer P17302,
- des Hyaluronsäure-Rezeptors CD44 mit der Swiss-Prot-Nummer P16070,
- der Fatty Acid Synthase (FAS) mit der Enzymklassifizierung EC 2.3.1.85 und der Swiss-Prot-Nummer P49327,
- des Epidermal Fatty Acid Binding Proteins FABE mit der Swiss-Prot-Nummer Q01469,
- des Proteins PSMD2 mit der Swiss-Prot-Nummer Q13200 und
- des DNA binding protein A (DBPA) mit der Swiss-Prot-Nummer P16989
in Zellen der Haut und der Hautanhangsgebilde.

6. Nicht-therapeutische, kosmetische topische Verwendung von Apfelkernextrakten zur Inhibierung der Expression des Bone Proteoglycan II Precursors (PGS2) mit der Swiss-Prot-Nummer P07585 in Zellen der Haut und der Hautanhangsgebilde.

## Claims

1. Non-therapeutic, cosmetic topical use of apple seed extracts for increasing the intercellular cell adhesion in the skin and the skin appendages.

2. Non-therapeutic, cosmetic topical use of apple seed extracts for increasing the adhesion between cells and the extracellular matrix in the skin and the skin appendages.

3. Non-therapeutic, cosmetic topical use of apple seed extracts for increasing the lipid production in the epidermis.

4. Non-therapeutic, cosmetic topical use of apple seed extracts for increasing the formation of intercellular communication pores in the skin and the skin appendages and for improving the intercellular cell communication.

5. Non-therapeutic, cosmetic topical use of apple seed extracts for increasing the expression
- of the keratin 5 (KRT5) having the Swiss-Prot number P13647,
- of the keratin 14 (KRT14) having the Swiss-Prot number P02533,
- of the keratin 10 (KRT10) having the Swiss-Prot number P13645,
- of the keratin 1 (KRT1) having the Swiss-Prot number P04264,
- of the gap junction protein beta 2 connexin 26 (CXB 2) having the Swiss-Prot number P29033,
- of the gap junction protein alpha 1 connexin 43 having the Swiss-Prot number P17302,
- of the hyaluronic acid receptor CD44 having the Swiss-Prot number P16070,
- of the fatty acid synthase (FAS) having the enzyme classification EC 2.3.1.85 and the Swiss-Prot number P49327,
- of the epidermal fatty acid binding protein FABE having the Swiss-Prot number Q01469,
- of the protein PSMD2 having the Swiss-Prot number Q13200 and
- of the DNA binding protein A (DBPA) having the Swiss-Prot number P16989,
in cells of the skin and the skin appendages.

6. Non-therapeutic, cosmetic topical use of apple seed extracts for inhibiting the expression of the bone proteoglycan II precursor (PGS2) having the Swiss-Prot number P07585 in cells of the skin and the skin appendages.

## Revendications

1. Utilisation cosmétique topique, non thérapeutique d'extraits de pépins de pomme pour augmenter l'adhésion intercellulaire des cellules dans la peau et les phanères.

2. Utilisation cosmétique topique, non thérapeutique d'extraits de pépins de pomme pour augmenter l'adhésion entre des cellules et la matrice extracellulaire dans la peau et les phanères.

3. Utilisation cosmétique topique, non thérapeutique d'extraits de pépins de pomme pour augmenter la production lipidique dans l'épiderme.

4. Utilisation cosmétique topique, non thérapeutique d'extraits de pépins de pomme pour augmenter la formation de pores de communication intercellulaire dans la peau et dans les phanères et pour améliorer la communication intercellulaire des cellules.

5. Utilisation cosmétique topique, non thérapeutique d'extraits de pépins de pomme pour augmenter l'expression
- de la kératine 5 (KRT5) portant le numéro Swiss-Prot P13647 ;
- de la kératine 14 (KRT14) portant le numéro Swiss-Prot P02533 ;
- de la kératine 10 (KRT10) portant le numéro Swiss-Prot P13645 ;
- de la kératine 1 (KRT1) portant le numéro Swiss-Prot P04264 ;
- de la protéine de jonction communicante, la connexine 26 bêta 2, (CXB2) portant le numéro Swiss-Prot P29033 ;
- de la protéine de jonction communicante, la connexine 43 alpha 1, portant le numéro Swiss-Prot P17302 ;
- du récepteur CD44 de l'acide hyaluronique portant le numéro Swiss-Prot P16070 ;
- de la synthase d'acide gras (FAS) possédant le numéro de classification des enzymes EC 2.3.1.85 et le numéro Swiss-Prot P49327 ;
- de la protéine de liaison d'acide gras épidermique FABE portant le numéro Swiss-Prot Q01469 ;
- de la protéine PSMD2 portant le numéro Swiss-Prot Q13200 ; et
- de la protéine A de liaison à l'ADN (DBPA) portant le numéro Swiss-Prot Q 16989
dans les cellules de la peau et des phanères.

6. Utilisation cosmétique topique, non thérapeutique d'extraits de pépins de pomme pour inhiber l'expression du précurseur II des protéoglycanes de l'os (PGS2) portant le numéro Swiss-Prot P07585 dans les cellules de la peau et des phanères.
